# EUROPEAN PATENT APPLICATION

(11) **EP 1 615 033 A1**
(43) Date of publication of application: **11.01.2006**
(21) Application number: 04722949.7
(22) Date of filing: 24.03.2004
(51) Int. Cl.: G01N 33/50, G01N 33/15, C12Q 1/02

(54) **METHOD OF SCREENING SUBSTANCE INTERACTING WITH ABC PROTEIN**

(30) Priority: 25.03.2003 JP 2003083686
(71) Applicant: The Circle for the Promotion of Science and Engineering, Tokyo 152-8550 (JP)
(72) Inventor: YABUUCHI, Hikaru, GenoMembrane, Inc., Yokohama-shi, Kanagawa 230-0046 (JP); ISHIKAWA, Toshihisa, Tokyo Inst. of Technology, Yokohama-shi, Kanagawa 226-8502 (JP)
(74) Representative: van Westenbrugge, Andries
(86) International application number: PCT/JP2004/004093
(87) International publication number: WO 2004/086036

(57) **Abstract**

The present invention provides the following: a measuring method for transporter activity of ABC protein that can measure not only small amount of samples with small data fluctuation and good reproducibility, but also many samples simultaneously and concisely; a screening method for substances that interact with ABC protein using said measuring method; and a kit for measuring transporter activity of ABC protein that can be used advantageously for those methods. Pre-incubation is carried out by adding the insect cellular membrane expressing human P-glycoprotein to the reactionbuffer solution including vanadic acid and [³H] ATP. Following this, the test compound is added for reaction, after which reaction liquid is added to a glass filter of 96-well type, and by using the suction cleaning method, adsorption amount of [³H] ADP into the membrane-expressing P-glycoprotein is measured through 1 step.

## Description

### Technical Field

The present invention relates to a screening method for substances that interact with ABC (ATP Binding Cassette) protein (ABC transporter), a measuring method for transporter activity of ABC protein, and a kit for measuring transporter activity of ABC protein that can be used advantageously for those screening and measuring methods.

### Background Art

By transporting ion and nutrient as well as eliminating waste products and toxin through such intracellular organelle membranes as plasma membrane, endoplasmic reticula, and mitochondrion, it is possible for the cells to maintain cellular function. These material transportations are selectively carried out by membrane transport protein called channel or transporter. There are some known mechanics enabling substances like ions or nutrients to penetrate membranes, and ABC protein is known as one of those mechanisms. (See C. F. Higgins, Ann. Rev. Cell Biol., 8, 67 (1992) for example). ABC protein is differentiated into such various functions as transporter, channel, and receptor (regulator), playing an important physiological function in each organism, each having similar secondary structure, a membrane protein family having ATP binding domain comprising transmembrane domain in common, and is driven and controlled by intracellular ATP and ADP. ABC protein is one of the biggest gene families spreading in a wide range of organisms from bacteria, yeast, plants, to mammals. Nowadays, since more than 50 ABC protein genes are identified in human, most of which are related to active transfer of intermembranous molecules, ABC protein is called ABC transporter.

Since ABC protein transforms the energy of ATP hydrolysis into conformational change of protein molecule, and transfers drugs extracellularly by coupling with it, it regulates the disposition profile of drugs (drug effective concentration in absorption, distribution, metabolism, excretion, site on target), which in turn determines the total pharmacological effect of drugs. For example, ABC transporter, which was expressed in intestinal epithelial cells and cerebrovascular endothelial cells, has a great influence on the bioavailability of orally administered drugs and drug migration to central nervous system. Moreover, the disorder of ABC protein genes is said to be related with the diseases, particularly in human, it has become clear that the disorder of ABC protein is responsible for various diseases, and the importance of biological defense mechanism of ABC protein has begun to be understood. For example, when P-glycoprotein (MDR1), which belongs to ABC protein family, and MRP1, are overexpressed, it is well known in the field of chemical therapy of cancer that cancerous cells become tolerant by excreting many anti-cancerous drugs out of the cells.

The mechanism of transporting drugs by ABC protein as a transporter via cellular membrane is considered as follows:
(1) ABC protein is present in both sides (intracellular side and extracellular side) of the cellular membrane.
(2) One molecule of ATP as the source of energy as well as 1 molecule of drugs which is supposed to be transported is bound to the ABC protein intracellularly, whereas ATP is hydrolyzed into ADP and phosphate by ATPase activity of ABC protein, the drugs are transported from inside to outside of cells through transformation of ABC protein caused by the energy emitted at the hydrolysis.
(3) Along with the extracellular emission of the drugs, biosynthesized phosphate in (2) is also released from the ABC protein.
(4) A new ATP molecule is bound to ABC protein from inside of cells and this ATP is dissolved into ADP and phosphate, and the energy, by which the formation of ABC protein transformed in (2) is recovered, is released.
(5) ADP and phosphate which are produced in said (4) are released from ABC protein, and the transport system goes back to the condition of said (1).

Nowadays, in an assay of the most distinguished P-glycoprotein among ABC transporters, many pharmaceutical companies, etc., adopt the measuring method for ATPase activity when substrate is addedto the membrane expressing P-glycoprotein. Specifically, since ATP is bound to ABC protein and dissolved into ADP in the above cycle by the presence of drugs which are supposed to be transported in the stage of above (2), drugs which are recognized by ABC protein can be detected if ADP is measured under the condition of being bound to ABC protein. Therefore, drugs or inhibitors transported by ABC protein can be detected if labeling is added to ATP, subsequently ABC protein, which is bound to the labeling (that is to say, ABC protein bound to labeled ADP), is measured. This method has such merits as the very cheap price of reagent or the ability to handle a large amount of samples. However, in fact, it is difficult to measure labeled ABC protein because the above transport system is in the dynamic condition of enzymatic rotation. Moreover, since the background becomes a little higher by endogenous ATPase activity of the membrane itself, and since degradation amount of phosphate concentration is measured, there is a demerit in its relatively low sensitivity.

Instead of the measuring method for ATPase activity mentioned above, an assay which was adopted from Vanadate Method was contrived. Vanadate Method is the method which has been reported in the papers since 1995, and it uses the phonemenon of binding between ADP and transporter caused by the substitution of phosphate ion with vanadate, occurred at the dissolution of ATP by ABC transporter. That is to say, if ATP, which was labeled in advance with radio isotope etc., is used as ATP, and if vanadic acid becomes present in the transport system mentioned above, vanadic acid is substituted with phosphate acid in the stage of (2) mentioned above, the rotation of the above transport system stops, the labeled ADP stays in the condition of being bound to ABC protein, the quantity of ADP, being bound to ABC protein, can be determined, thus, it becomes possible to measure transporter activity of ABC protein indirectly.

In this measurement, conventionally, membrane protein and ABC protein were isolated from cellular membrane, the isolated ABCproteinwas separated from other proteins by electrophoresis, and the labeling, which is bound to ABC protein (that is to say, labeled ADP), has been measured. According to this method, it was possible to determine whether the test drugs are the substrates of ABC protein or inhibitors whereas it was impossible to examine a great amount of test articles in a short time.

Thus, the inventors have suggested a screening method for substrate of ABC protein comprising the steps of: screening method for substrate of ABC Binding cassette (ABC) protein, wherein cell membrane fraction expressing ABC protein, labeled ATP and vanadic acid as well as the test samples are mixed and incubated, this mixture is added to the base in which antibody against ABC protein is immobilized, or this mixture is mixed with labeling reagent and test sample etc. and incubated on the base, then the immobilized labeling or labeling without immobilization is measured (See International Publication WO02/052262 pamphlet). According to the screening method, by binding membrane fraction expressing ABC transporter to the base via antibody (for example 96-well microplate), and by measuring the binding amount of [³²P] ATP (ADP) bound to ABC transporter, it becomes possible for substrate or inhibitor against ABC transporter to be screened quite effectively and to apply for high-through put screening. However, in the above method and kit, since the biological activity of membrane fraction, expressing ABC transporter after being immobilized to the base via antibody is unstable, and since there is such a problem in the reproducibility of experimental data by limiting the amount of membrane fraction adsorbable per well, it became indispensable to formulate more practical experimental system that is possible for stable data analysis.

The task of the present invention is to provide the following: a measuring method for transporter activity of ABC protein that can measure not only small amount of samples with small data variation and good reproducibility, but also many samples simultaneously and concisely; a screening method for substances that interact with ABC protein using said measuring method; and a kit for measuring transporter activity of ABC protein that can be used advantageously for those methods.

In order to solve the above task, the present inventors have carried out a keen research, and have started to improve the existing Vanadate Method (See International Publication WO02/052262 pamphlet). Pre-incubation is carried out by adding the insect cellular membrane expressing human P-glycoprotein to the reaction buffer solution including vanadic acid and [³H] ATP. Following this, the test compound is added for reaction, after which reaction liquid is added to a glass filter of 96-well type, and by using the suction cleaning method, adsorption amount of [³H] ADP into P-glycoprotein-expressing membrane is measured through one step. As a result of this, it has been found out that it is possible to measure not only small amount of samples with small data variation and good reproducibility, but also many samples simultaneously and concisely. Thus, the present invention has been completed.

### Disclosure of the Invention

Therefore, the present invention is related to a screening method for a substance that interacts with ABC (ATP Binding Cassette) protein, wherein it has the following processes: a process of contacting amembrane fractionexpressingABCprotein, labeled nucleoside triphosphate or its derivative, nucleoside diphosphate immobilized substance which forms a complex with nucleoside diphosphate by substituting phosphate ion of nucleoside triphosphate or its derivative and maintains the binding between nucleoside diphosphate and membrane protein and a test article; a process of cleaning the membrane fraction expressing the membrane protein with cleaning solvent, and separating at least unreacted lalbeled nucleoside triphosphate or its derivative by filtration through a filter; a process of measuring the labeling on the filter and/or the labeling in the filtrate ("1"), the screening method for a substance that interacts with ABC protein according to "1", wherein the process of separating by filtration through a filter is a separating process of suction filtration and/or centrifugal filtration through a filter ("2"), the screening method for a substance that interacts with ABC protein according to "1" or "2", wherein the nucleoside biphosphate immobilized substance is vanadic acid ("3"), the screening method for a substance that interacts with ABC protein according to any one of "1" to "3", wherein the nucleoside triphosphate is ATP ("4"), the screening method for a substance that interacts with ABC protein according to any one of "1" to "4", wherein the labeling is radioactive labeling, fluorescent labeling or photoaffinity group labeling ("5"), the screening method for a substance that interacts with ABC protein according to "5", wherein the radioactive labeling is ³²P, ³³P, ³⁵S, ¹⁴C, or ³H ("6"), the screening method for a substance that interacts with ABC protein according to any one of "1" to "6", wherein the membrane fraction expressing ABC protein is a membrane fraction of a mammal or an insect expressing ABC protein ("7"), the screening method for a substance that interacts with ABC protein according to any one of "1" to "7", wherein the substance that interacts with ABC protein is a substrate for ABC protein ("8"), the screening method for a substance that interacts with ABC protein according to any one of "1" to "7", wherein the substance that interacts with ABC protein is an inhibitor of ABC protein ("9"), and the screening method for a substance that interacts with ABC protein according to any one of "1" to "9", wherein the ABC protein is a human ABC protein which belongs to ABCA subfamily, ABCB subfamily, ABCC subfamily, ABCD subfamily, ABCE subfamily, ABCF subfamily, or ABCG subfamily ("10").

The present invention is also related to a kit for measuring transporter activity of ABC (ATP Binding Cassette) protein, having a filter which can filtrate and separate a membrane fraction expressing ABC protein, labeled nucleoside triphosphate or its derivative, and nucleoside diphosphate immobilized substance which forms a complex with nucleoside disphosphate by substituting phosphate ion of nucleoside triphosphate or its derivative and maintains the binding between nucleoside diphosphate and membrane protein, and at least unreacted labled nucleoside triphosphate or its derivative ("11"), the kit for measuring transporter activity of ABC protein according to "11", wherein the filter which can filtrate and separate is equipped with the means of suction filtration and/or centrifugal filtration ("12"), the kit for measuring transporter activity of ABC protein according to "11" or "12", wherein the nucleoside diphosphate immobilized substance is vanadic acid ("13"), the kit for measuring transporter activity of ABC protein according to any one of "11" to "13", wherein the nucleoside triphosphate is ATP ("14"), the kit for measuring transporter activity of ABC protein according to any one of "11" to "14", wherein the labeling is radioactive labeling, fluorescent labeling or photoaffinity group labeling ("15"), the kit for measuring transporter activity of ABC protein according to "15", wherein the radioactive labeling is ³²P, ³³P, ³⁵S, ¹⁴C, or ³H ("16"), the kit for measuring transporter activity of ABC protein according to any one of "11" to "16", wherein the membrane fraction expressing ABC protein is a membrane fraction of a mammal or an insect expressing ABC protein ("17"), the kit for measuring transporter activity of ABC protein according to any one of "11" to "17", wherein the substance that interacts with ABC protein is a substrate for ABC protein ("18"), the kit for measuring transporter activity of ABC protein according to any one of "11" to "17", wherein the substance that interacts with ABC protein is an inhibitor of ABC protein ("19"), and the kit for measuring transporter activity of ABC protein according to any one of "11" to "19", wherein the ABC protein is a human ABC protein which belongs to ABCA subfamily, ABCB subfamily, ABCC subfamily, ABCD subfamily, ABCE subfamily, ABCF subfamily, or ABCG subfamily ("20").

The present invention is further related to a measuring method for transporter activity of ABC (ATP Binding Cassette) protein, wherein it has the following processes: a process of contacting a membrane fraction expressing ABC protein, labeled nucleoside triphosphate or its derivative, and nucleoside diphosphate immobilized substance which forms a complex with nucleoside diphosphate by substituting phosphate ion of nucleoside triphosphate or its derivative and maintains the binding between nucleoside diphosphate and membrane protein, and a substrate of ABC protein; a process of cleaning the membrane fraction expressing the membrane protein with cleaning solvent, and separating at least unreacted labeled nucleoside triphosphate or its derivative by filtration through a filter; a process of measuring the labeling on the filter and/or the labeling in the filtrate ("21").

### Brief Description of Drawings

Fig. 1 is a schematic drawing showing time course of [³H] ATP-binding activity in the following situation: when verapamil is added to P-glycoprotein-expressing membrane (• :P-gp/Ver. (+)), when verapamil is not added to P-glycoprotein-expressing membrane (o :P-gp/Ver. (-)), when verapamil is added to Sf9 control membrane (■ : Control/Ver. (+)), and when verapamil is not added to Sf9 control membrane (□ : Control/Ver. (+)).
Fig. 2 is an analyzing drawing of the usage of P-glycoprotein--expressing membrane that shows changes of [³H] ATP-binding activity in the following situation: when verpamil is added to 5 µg, 2.5 µg, or 1 µg of P-glycoprotein-expressing membrane (Verapamil (+)), or when not added (Verapamil (-)).
Fig. 3 is a schematic drawing showing concentration dependency of verapamil upon P-glycoprotein.
Fig. 4 is a schematic drawing showing binding activity of various test articles to P-glycoprotein.
Fig. 5 is a schematic drawing showing the result of study on cleaning condition in the following situation: the combination of cleaning with reaction buffer solution A and blocking method by PVP (Buf. A + PVP), cleaning with reaction buffer solution A (Buf. A), the combination of cleaning with PBS and blocking method by PVP (PBS + PVP), and cleaning with PBS (PBS).
Fig. 6 is a schematic drawing showing measurement results of [³H] ADP-binding amount to human MRP2-expressing Sf9 membrane by vanadate method when adding A; β-estradiol-17-(β-D-glucuronide) and B; Sulfobromophthalein.

### Best Mode of Carrying Out the Invention

As for the screening method for substances that interact with ABC protein of the present invention, it should not be particularly restricted as long as the screening method includes the following processes: a process of contacting a membrane fraction expressing ABC protein, labeled nucleoside triphosphate or its derivative, and nucleoside diphosphate immobilized substance which forms a complex with nucleoside diphosphate by substituting phosphate ion of nucleoside triphosphate or its derivative and maintains the binding between nucleoside diphosphate and membrane protein and a test article; a process of cleaning the membrane fraction expressing the membrane protein with cleaning solvent, and separating at least unreacted labeled nucleoside triphosphate or its derivative by filtration through a filter; a process of measuring the labeling on the filter and/or the labeling in the filtrate. Also, as for the measuring method for transporter activity of ABC protein of the present invention, it should not be particularly restricted as long as the measuringmethod contains the following processes: a process of contacting a membrane fraction expressing ABC protein, labeled nucleoside triphosphate or its derivative, and nucleoside diphosphate immobilized substance which forms a complex with nucleoside diphosphate by substituting phosphate ion of nucleoside triphosphate or its derivative and maintains the binding between nucleoside diphosphate and membrane protein and a substrate of ABC protein; a process of cleaning the membrane fraction expressing the membrane protein with cleaning solvent, and separating at least unreacted labeled nucleoside triphosphate or its derivative by filtration through a filter; a process of measuring the labeling on the filter and/or the labeling in the filtrate. Moreover, as for the kit for measuring transporter activity of ABC protein of the present invention, it should not be particularly restricted as long as the kit for measuring transporter activity of ABC protein has a filter which can filtrate and separate a membrane fraction expressing ABC protein, labeled nucleoside triphosphate or its derivative, and nucleoside diphosphate immobilized substance which forms a complex with nucleoside disphosphate by substituting phosphate ion of nucleoside triphosphate or its derivative and maintains the binding between nucleoside disphosphate and membrane protein and at least unreacted labeled nucleoside triphosphate or its derivative.

ABC protein transports substances with the energetic power obtained by hydrolysis of ATP as a driving force, and controls the channels. ABC protein has characteristic ATP-binding site, whose primary structure is maintained beyond kinds of ABC protein, species of organisms, and through evolution. Particularly, ATP-binding site of ABC protein is conserved with amino acid residue sequence compared to the primary structure of the transmembrane part. Walker A and Walker B motif (Walker, J. E., Saraste, M., Runswick, M. J., Gay, N. J. (1982) EMBO J 1, 945-941) and ABC signaturemotif (Higgins, C. F. (1992) Annu. Rev. Cell Biol. 8, 67-113) are present almost without exception in the ATP binding site as the common amino acid residue sequence.

In the present invention, ABC protein means a protein which contains Walker A motif (Gly-X-X-Gly-X-Gly-Lys-Ser-[Ser, Thr, Gln] (SEQ ID NO: 1) and Walker B motif ([Leu, Ile, Phe]-[Ile, Leu, Val] -X-Asp- [Glu, Asp, Ser]) (SEQ ID NO: 2) and the following ABC signature motif: ([Leu, Ile, Val, Met, Phe, Tyr, Cys] - [Ser, Ala]-[Ser, Ala, Pro, Gly, Leu, Val, Phe, Tyr, Lys, Gln, His]-Gly-[Asp, Glu, Asn, Gln, Met, Trp]-[Lys, Arg, Gln, Ala, Ser, Pro, Cys, Leu, Ile, Met, Phe, Trp]-[Lys, Arg, Asn, Gln, Ser, Thr, Ala, Val, Met]-[Leu, Ile, Val, Met, Phe, Tyr, Pro, Ala, Asn] - [Pro, His, Tyr] - [Leu, Ile, Val, Met, Phe, Trp] - [Ser, Ala, Gly, Cys, Leu, Ile, Val, Phe] - [Phe, Tyr, Trp, His, Pro] - [Lys, Arg, His, Pro]-[Leu, Ile, Val, Met, Phe, Tyr, Trp, Ser, Thr, Ala] (however, X stands for unparticular amino acid) (Sequence No. 3) and similar proteins thereof, and their origins include bacteria, yeast, plants, animals, but not particularly limited thereto.

As for the ABC protein, the following proteins can be pointed out: ABCA1 (code name ABC1, TGD, HDLDT1), ABCA2 (code name: ABC2), ABCA3 (code name: ABC3, ABC-C, EST111653), ABCA4 (code name: STGD1, RP19, FEM, STGD), ABCA5 (code name: EST90625), ABCA6 (code name: EST155051), ABCA7, ABCA8, ABCA9, ABCA10 (code name: EST698739), ABCA11 (code name: EST1133530), ABCA12(DKFZP434G232) etc., which are human ABC proteins that belong to ABCA subfamily (code name: ABC1 subfamily); ABCB1 (code name: MDR1, P-gp, PGY1), TAP1 (code name: PSF1, RING4, D6S114E), TAP2 (code name: PSF2, RING11, D6S217E), ABCB4 (code name: MDR2/3, PFIC-3, PGY3), ABCB5 (code name: EST422562), ABCB6 (code name: EST45597, umat, Hs. 107911), ABCB7 (code name: EST140535, Atmlp, ABC7), ABCB8 (code name: EST328128, M-ABC1), ABCB9 (code name: EST122234), ABCB10 (code name: EST20237), ABCB10P (code name:M-ABC2, MABC2), ABCB11 (code name: SPGP, PFIC-2, BSEP, PGY4), etc., which are human ABC proteins that belong to ABCB subfamily (code name: MDR/TAP subfamily); ABCC1 (code name: MRP1, MRP, GS-X), ABCC2 (code name: MRP2, cMOAT, cMRP), ABCC3 (code name: MRP3, cMOAT2, EST90757, MLP2, MOAT-D, ABCC4 (code name: MRP4, EST170205, MOAT-B), ABCC5 (code name: MRP5, SMRP, EST277145, MOAT-C), ABCC6 (code name: MRP6, EST349056, MLP1, ARA), CFTR (code name: CF, MRP7, ABCC 7), ABCC8 (code name: SUR1, MRP8, PHHI, HI, HRINS), ABCC9 (code name: SUR2), ABCC10 (code name: EST182763), ABCC11, ABCC12 (code name: MRP9), ABCC13 (code name: PRED6, C21orf73)etc., which are human ABC proteins that belong to ABCC subfamily (code name: CFTR/MRP subfamily) ; ABCD1 (code name: ALDP, ALD, AMN) , ABCD1P1, ABCD1P2, ABCD1P3, ABCD1P4, ABCD2 (code name: ALDR, ALDRP, ALDL1), ABCD3 (code name: PM70, PXMP1), ABCD4 (code name: P70R, EST352188, PXMP1L, PMP69) etc., which are ABC proteins that belong to ABCD subfamily (code name: ALD subfamily); ABCE1 (code name: RNS4I, RL1,OABP, RNASELI) etc., which are ABC proteins that belong to ABCE subfamily (code name: OABP subfamily) ; ABCF1 (codename: EST123147, ABC50), ABCF2 (code name: EST133090, Hs. 153612), ABCF3 (code name: EST201864) etc., which are human ABC proteins that belong to ABCF subfamily (code name: GCN20 subfamily); ABCG1 (code name: WHITE, ABC8), ABCG2 (code name: EST157481, MXR, BCRP, ABCP), ABCG3 (code name: Abcp2, Mxr2), ABCG4 (code name: WHITE2), ABCG5, ABCG8, etc., which are human ABC proteins that belong to ABCG subfamily (code name: WHITE subfamily), etc. Among them, P-glycoprotein (P-gp), MRP1, MRP2, CFTR, SUR1, ABC1, TAP1 etc. may be preferably exemplified.

As for the above membrane fraction expressing ABC protein, any membrane fraction is acceptable as long as it is a fraction of a biological membrane into which ABC protein gene is introduced and in which ABC protein is expressed. As for the biological membrane, such intracellular organelle membranes as membrane, endoplasmic reticula, mitochondria may be pointed out. These biological membranes may be derived from the following animal and plant cells but are not particularly limited to them: bacterial prokaryotic cells such as E-coli, Streptomyces, Bacillus subtilis, Streptococcus, staphylococcus; eukaryotic cells such as yeast, Aspergillus; insect cells such as Drosophila S2, Spodoptera Sf9; animal cells such as L cell, CHO cell, COS cell, HeLa cell, C127 cell, BALB/c3T3 cell, BHK21 cell, HEK293 cell, VERO cell, CV-1 cell, MDCK cell, Bowes melanoma cell, oocyte of Xenopus. Among these, such insect cells as Drosophila S2, Spodoptera Sf9, etc. are preferable.

As for the preparation method for membrane fraction expressing ABC protein, the method for preparing membrane fractions from cells expressing ABC proteins is exemplified. As for the above preparation method for cells expressing ABC protein, for example, a method, in which cDNA encoding ABC protein is integrated into an expression vector having a selective marker, and the expression vector is transfected to cultured cells such as mammalian cells, insect cells, yeast and bacteria may be mentioned. Only cells having expression vector of ABC protein is selected by using a vector containing genes that add resistance to such particular antibiotics as neomycin, puromycin, hydromycin, and so on, as an expression vector having a selective marker, and by culturing transfected cells in the presence of antibiotics. It is preferable to confirm the expression of ABC protein by RT-PCR or specific antibody. The great amount of ABC protein-expressing cells which are obtained in such a way is cultured. Moreover, as for the preparation method for membrane fraction from cells expressing ABC protein, such separating and purification method of cell membrane fraction as the following can be pointed out: the culture of the above ABC protein-expressing cells is centrifuged, and cells expressing ABC protein are collected and fractured through ultrasound, osmotic pressure treatment, and so on. The membrane fraction is then extracted by centrifugation, and the extracted membrane fraction is suspended in the buffer solution by sucrose concentration-gradient centrifugation. It is preferable for the prepared membrane fraction to be frozen instantly in the temperature of liquid nitrogen after measuring the protein concentration, and to be kept at -80°C.

As for the labeling in labeled nucleoside triphosphate or its derivative, the following labeling may be pointed out although it should not be limited particularly as long as it is detectable: radioactive labeling using such radio isotopes as ³²P, ³³P, ³⁵S, ¹⁴C, ³H; fluorescent labeling using fluorescein isothiocyanate, phycobiliprotein, rare-earth metal chelate, dansyl chloride, tetramethylrhodamine isothiocyanate, etc.; photoaffinity labeling using azido group, benzophonone, etc.; enzymatic labeling using peroxidase, alkaline phosphatase, etc. Among these, it is particularly preferable to exemplify radioactive labeling.

As for the nucleoside triphosphate in labeled nucleoside triphosphate or its derivatives, ATP, GTP, ITP, UTP, CTP, etc., may be exemplified. Among them, it is particularly preferable to exemplify ATP. Also, as for such derivative of nucleoside triphosphate, for example, as the derivative of ATP, [³⁵S] ATPyS, α, β-methylene ATP, 8-azido ATP, may be pointed out.

As for the nucleoside disphosphate immobilized substance of the present invention, it should not be particularly limited as long as it is the chemical substance which forms a complex with nucleoside diphosphate by substituting phosphate ion of nucleoside triphosphate or its derivative and maintains the binding between nucleotide diphosphate and membrane fraction. Such examples as vanadic acid and its salt may be pointed out. As for the salt of vanadic acid, the following may be exemplified: sodium vanadate (sodium orthovanadate Na₃VO₄, sodium pyrovanadate Na₄V₂O₇, sodium methavanadate NaVO₃), potassium vanadate (potassium orthovanadate K₃VO₄, potassium pyrovanadate K₄V₂O₇, potassium methavanadate KVO₃), calcium vanadate (calcium orthovanadate Ca₃ (VO₄)₂, calcium pyrovanadate Ca₂V₂O₇, calcium methavanadate Ca(VO₃)₂).

As for the filter used in the present invention, it should not be limited particularly as long as it is the filter which may filtrate and separate unreacted labeled nucleoside triphosphate or its derivatives. Yet, it is preferable, in the view of efficiency of filtration and separation, to be equipped with suction filtration means, centrifugual filtration means, ultrafiltration means and so on. Among them, it is preferable for the plate to include a filter at its bottom, or to be equipped with suction filtration means at the lower part thereof. For example, the microplate equipped with a filter at its bottom named, "UNIFILTER" (No. 7700-3303, Whatman Co.), MultiScreen Plate (MILLIPORE CO.), Silent Screen Plate (Nalge Nunc Co.), etc., may be specifically pointed out.

As for the test articles in the screening method for substances that interact with ABC protein of the present invention, it is preferable to exemplify nominated substance of such drugs as antitumor medicine. Also, as for the substrate of ABC protein in the measuring method for transporter activity of ABC protein in the present invention, when ABC protein is P-glycoprotein, such some steroids as verapamil (Vasolan), quinidine, rhodamine 123, doxorubicin, digoxin, vincristine, vinblastine, daunorubicin, folate, cortisol, aldosteronmaybe pointed out. β-estradiol-17- (β-D-glucuronide), sulfobromophthalein, etc., may be pointed out when ABC protein is MRP2.

As for the screening method for substances that interact with ABC protein in the present invention [measuring method for transporter activity of ABC protein], a case wherein ABC protein is P-glycoprotein, labeled nucleoside triphosphate is radioactively labeled ATP, and nucleoside diphosphate immobilized substance is vanadic acid, will be explained. Membrane fraction expressing P-glycoprotein, radioactive labeled ATP, vanadic acid, and test article [substrate of P-glycoprotein] are reacted in reaction buffer solutions such as Tris-HCl and phosphate buffer solution. It is preferable for the pH of reaction buffer solution to be 6 to 8, particularly from 7 to 7.5. It may be desirable for the reaction time to be from 10 seconds to 3 hours, for example, around 1 to 30 minutes. The reaction is stopped by adding excessive amount of buffer solution, which has been cooled off at for example 0°C, to reaction buffer (for example, 20 to 100 times as much amount as reaction liquid) and mixing the resultant solution. Following this, reaction liquid after the reaction is added to, for example, 96-well glass filter and suction cleaning is carried out. As for the cleaning solvent, buffer solution, which has been ice-cooled at 4°C in advance, is used, and suction cleaning is practiced for several times with the cleaning solvent of 50 to 500 µl per well, for example, 200 µl. Through this suction cleaning operation, unreactedATP, vanadic acid, and test article [substrate of P-glycoprotein] in the reaction liquid are filtrated,separated,and removed by the filter. Followingthis, radioactive labeling is measured with the use of liquid scintillation or β-counter, after adding 30 to 100 µl, for example 50 µl of liquid scintillation per well of the plate after cleaning, with membrane fraction, and leaving at rest for 1 hour at room temperature. Through the comparison between this measured value and the measured value in the case when test article [substrate of P-glycoprotein] is not added, it can be judged to be the substrate against P-glycoprotein when the transporter activity of P-glycoprotein is larger, whereas it may be determined to be the inhibitor of P-glycoprotein when the transporter activity of P-glycoprotein is smaller.

### [Examples]

The present invention will now be described in more detail with the examples below, but the technical scope of the invention shall not be limited to these examples.

### Example 1

### (Resource and Material)

By using the glass filter of 96-well type and suction cleaning method, the formulation of measuring method for [³H] ADP adsorption to P-glycoprotein-expressing membrane through one step has been challenged. As for the glass filter of 96-well type, 350 µl UNIFILTER (No. 7700-3303, Whatman Co.) was used. As for the reaction buffer solution A, the composition of 0.2 mM Na₃VO₄, 3 mM MgSO₄, 2 mM Ouabain, 0.1 mM EDTA, 0.05 mM ATP, 40 mM Tris-HCl (pH 7.4) was used. As for the radio isotope, [³H] ATP (NET-420, PerkinElmer Life Science Co.) was used. As for the Sf9 membrane, human P-glycoprotein (Pgp or MDR 1) expressing membrane (No. 453228, Daiichi Pure Chemicals Co.) and Sf9 control membrane (No. 453200, Daiichi Pure Chemicals Co., Ltd.) were used. As for the liquid scintillation cocktail, MicroScint (Registered Trademark) 20 (No. 6013621, Packard BioScience Co.) was used.

### (Measuring Method)

5 µg of P-glycoprotein-expressing membrane was added to 50 µl of the above reaction buffer solution A, and pre-incubation was practiced at 37°C for one minute. Following this, test articles [substrate compound of P-glycoprotein] were added to the above reaction liquid, and the resultant mixture was reacted for a particular time (1 to 30 minutes) at 37°C. After the reaction, the above reaction liquid was added to 96-well glass filter, and suction cleaning was practiced. The reaction buffer solution A, which had been ice-cooled in advance at 4°C, was used as cleaning liquid, and suction cleaning was carried out for 5 times with 200 µl per well of the cleaning solvent. After that, 50 µl of liquid scintillation cocktail was added per well of the plate after cleaning. After leaving the plate at rest for 1 hour at room temperature, RI activity was measured using TOP COUNT (C990201/42637) made by Packard Co. All the experiments were basically done with N = 3.

### Example 2 (Investigation of time course)

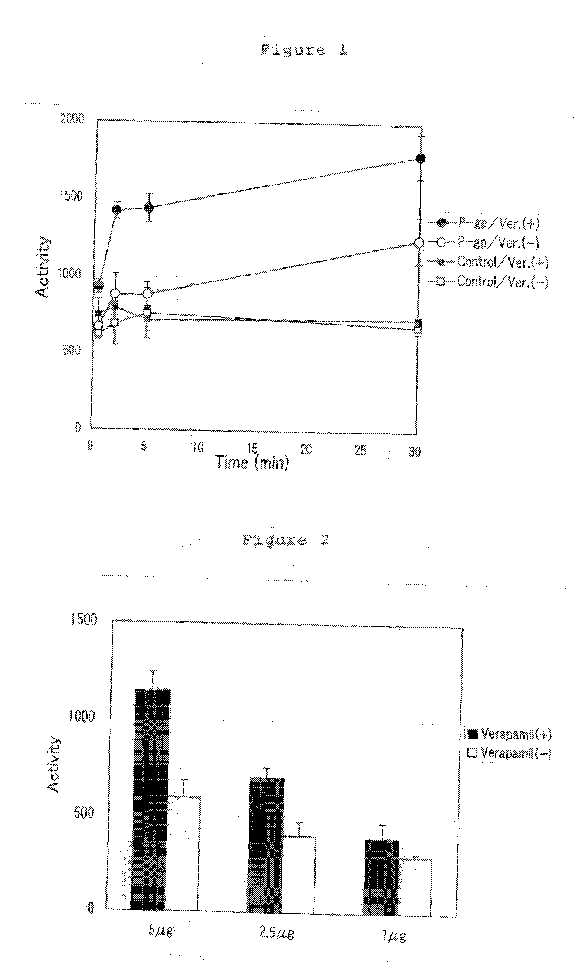

The time course of [³H] ADP binding amount by vanadate method was measured by using P-glycoprotein expressing membrane and Sf9 control membrane. Verapamil, which is known as the representative substrate of P-glycoprotein, was used as compound (positive control). The final concentration of verapamil was set at 50 µM. The result is shown in Fig. 1. From Fig. 1, in the control membrane, time course in the binding of [³H] ADP to the membrane was not seen, whereas in the P-glycoprotein-expressing membrane, it came to be understood that the increase of [³H] ADP binding amount was serially observed when verapamil was added. The interesting point is that some [³H] ADP-binding activity has been clearly observed, in other words, baselines became higher, even when verapamil was not added to P-glyocprotein-expressing membrane. This activity is considered to be the activity depending on the inherent activity of P-glycoprotein.

### Example 3 (Usage of Membrane)

The amount of reaction membrane per well can be set freely in the suction cleaning method using a glass filter. A reaction was conducted with verapamil as a compound, through 3 different stages of membrane amount per well (5 µg, 2.5 µg, 1 µg), in order to search for the optimal membrane amount for reaction. Concentration of added verapamil was set at 20 µM as its final concentration. The result is shown in Fig. 2. From Fig. 2, it became clear that the detection area expanded according to the increase of membrane usage. It was then determined that the appropriate membrane usage under this condition was 5 µg, and that the usage of P-glycoprotein-expressing membrane was set at 5 µg per well for the following experiments.

### Example 4 (Substrate Affinity of Verapamil and P-glycoprotein)

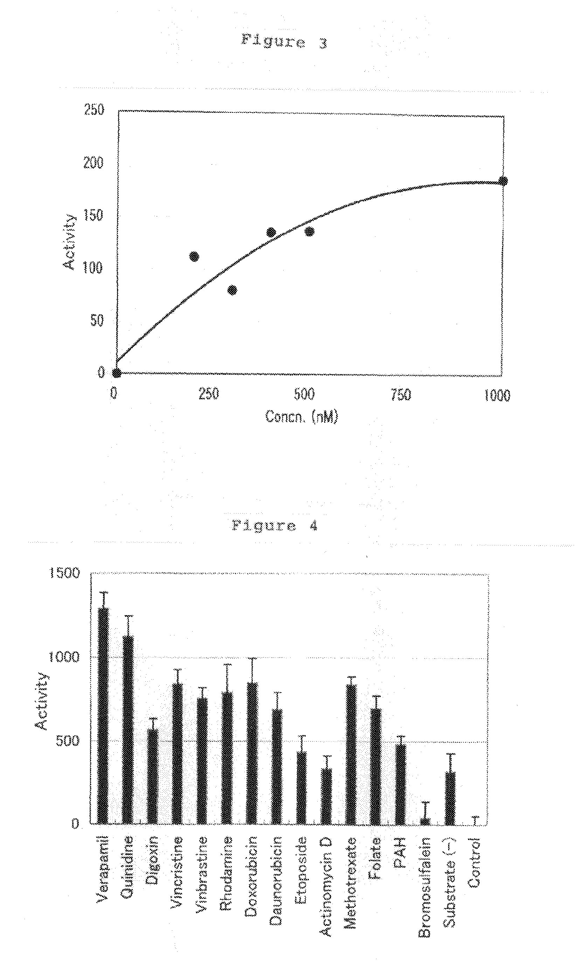

In order to measure substrate affinity (concentration dependency) of verapamil for P-glycoprotein, verapamil whose concentration is 0 to 1000 nM was addedto reactionbuffer solution A, and RI activity was measured. The result is shown in Fig. 3. As it can be seen from Fig. 3, Km value of about 300 nM was obtained. This is a very lowvalue, which is about 1/20 , compared to the conventional method where Km value of verapamil is 17 µM when ATPase activity of P-glycoprotein was measured. The reaction of ATP with P-glycoprotein is competitive when ATPase activity is measured, whereas in the vanadate method, once ATP (correctly speaking, ADP) is reacted, the apparent Km value was considered to be estimated lower because it is bound noncompetitively by vanadate. From this result, compared to the conventional ATPase method, it is considered to be possible for the assay method of the present invention to assay with low concentration.

### Example 5 (Substrate Specificity of P-glycoprotein)

Besides verapamil, [³H] ADP binding amount was measured through vanadate method in 13 kinds of compounds. The reaction condition was as follows; reaction time of 5 minutes, at reaction temperature of 37°C, and concentration of added substrate of 0.05 mM. In the measurement result, the background of control membrane is subtracted. The result is shown in Fig. 4. From Fig. 4, significant increase of [³H] ADP binding activity was observed in the following substrates which are generally considered to be the substrate of P-glycoprotein: Verapamil, Quinidine, Vinbrastine, Vincristine, Digoxin, Rhodamine123, Doxorubicin, Daunorubicin, Methotrexate, Folate. The interesting point is that in Bromosulfalein, it was observed that [³H] ADP binding activity lowered significantly, compared to the measurement value (Substrate (-)) obtained from P-glycoprotein-expressing membrane to which no substrate was added. This lowered value is considered to be the result of inhibiting the inherent activity of P-glycoprotein detected in Example 2, and this measurement value is almost equal to that of control membrane (Control). Bromosulfalein is generally considered to be the inhibitor of P-glycoprotein. Therefore, through the screening for compound using this assay system, it was found out that there is a probability of simultaneous detection whether it becomes the substrate of P-glycoprotein (agnoist) or its inhibitor (antagonist).

### Example 6 (Examination of Cleaning Method)

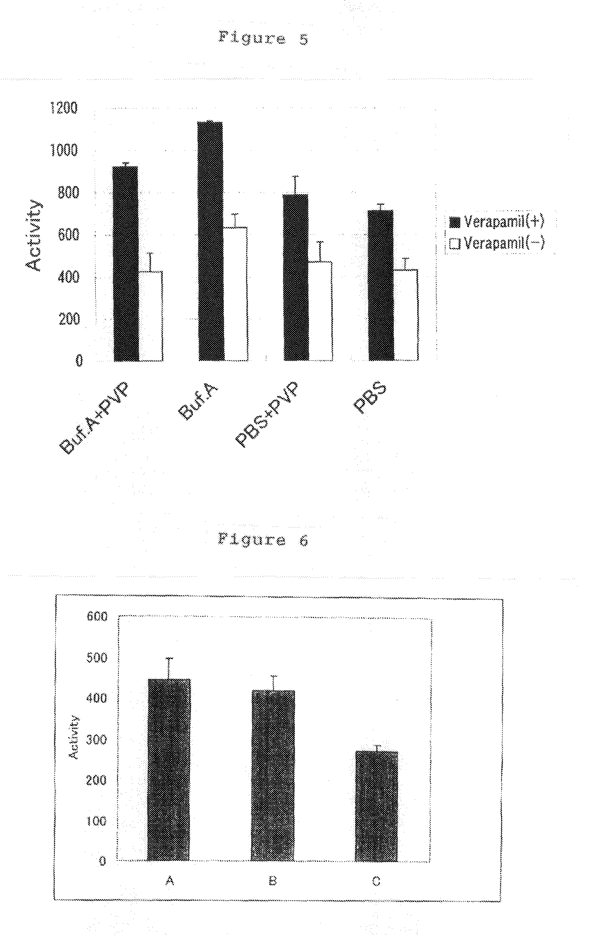

As a mean to lower the background of the measurement, method for blocking plates with PVP (polyvinyl pyrrolidone) and a method for cleaning plates with PBS, which are generally considered to be effective, have been analyzed. The final concentration of verapamil was set to be 20 µM. Specifically, the blocking method by PVP was conducted as follows: blocking solution prepared by dissolving 1% PVP in reaction buffer solution A, was added 200 µl per well right before the usage of the plate for cleaning, and where it was left for an hour at room temperature. Subsequently, it was used after cleaning once with 200 µl of reaction buffer solution A. Also, in the cleaning method by PBS, 200 µl per well was added while suction cleaning was done for 5 times. The result is shown in Fig. 5. As a result of this, any dramatic effect could be obtained from neither method. As for the cleaning by PBS, the specific binding of [³H] ADP was rather suppressed in the end. As for the cause of measurement background, it is considered that there is a great impact not only on the adsorption into the plate, but rather on the inherent binding to the membrane itself.

### Example 7 (Preparation of Human MRP2-expressing Sf9 membrane)

Through integrating cDNA encoding human MRP2, which had been cloned by ordinary method using PCR method, into Baculoviral vector, pFASTBacl (BAC-TO-BAC^{R} Baculovirus Expression Systems (Invitrogen, 10359-016)), Sf9 cell (Sf9 Frozen Cells B825-01, Invitrogen), was transfected by lipofection method. By recovering baculovirus from the transfected cells, cells expressing MRP2 protein were obtained through further infection of baculovirus to Sf9 cells. Also, by using serum-free insect media (SF900 II-SFM, Invitrogen), MRP2-expressing Sf9 cells, obtained by confirming the expression of MRP2 with the Western Blott Method, were cultured for 3 days at 27°C. After collecting MRP2-expressing Sf9 cells by centrifugation (3000 rpm for 10 minutes), they were fractured with sonification (treated for 10 minutes in the sonicator), and human MRP2-expressing Sf9 membrane fraction was extracted by centrifugation (100, 000 rpm for 30 minutes). The separated membrane fraction was then suspended in PBS buffer solution (pH 7.4), and the membrane fraction was isolated and purified with centrifugation in sucrose concentration gradient. After measuring protein concentration of membrane fraction, it was frozen instantly at the temperature of liquid nitrogen, and was kept at -80°C.

### Example 8

MRP2 is a kind of ABC transporter having a significant function in the detoxication mechanism of liver: it highly expresses in the biliary side of the liver, and exhausts many drugs from the liver to the large intestine side. The formulation of measuring method of [³H] ADP adsorption amount to human MRP2-expressing membrane was then attempted through one step of suction cleaning method by using glass filter of 96-well type. It was measured in the same way as Example 1 except for using human MRP 2-expressing Sf9 membrane prepared in Example 7 instead of human P-glycoprotein-expressing membrane. The binding amount of [³H] ADP was measured by vanadate method when β-estradiol-17 (β-D-glucuronide) and sulfobromophthalein, which are the representative compound well known for transporting to MRP2, were added. The reaction condition was as follows: reaction time for 5 minutes, reacting temperature at 37°C, at a concentration of added substrate of 0.1 mM. The result is shown in Fig. 6. In Fig. 6, A describes β-Estradiol-17-(β-D-glucronide), while B describes sulfobromophthalein, and C describes the case where substrate is not added. As a result of this, compared to the measurement value of MRP2-expressing membranes to which substrate is not added, the increase of significant [³H] ADP binding activity was observed in the two kinds of compounds mentioned above, whereas the utility of screening system by suction cleaning method was confirmed in MRP2.

### Industrial Applicability

As a result of condition study by suction cleaning method using the glass filter of the present invention, the establishment of P-glycoprotein and MRP2 detection system by vanadate method has been succeeded. Since the obtained measurement result shows small data fluctuation and good reproducibility, the present invention which is the applied method of vanadate method, has a very large merit in the substrate recognition measurement of such ABC proteins as P-glycoprotein and MRP2. Particularly, the following 4 merits can be pointed out when it is compared to vanadate method using ELISA plate disclosed in the International Publication WO02/052262 pamphlet:
1. The amount of membrane per well can be increased freely.
2. Since membrane binding step to ELISA plate can be cut, it is easy to control the quality of the products and is possible to lower its cost.
3. It is possible to assay with small amount of compound compared to conventional ATPase method.
4. It is possible to assay simultaneously whether the compound for assay is an agonist or antagonist to ABC transporter.

## Claims

1. A screening method for a substance that interacts with ABC (ATP Binding Cassette) protein, wherein it has the following processes: a process of contacting a membrane fraction expressing ABC protein, labeled nucleoside triphosphate or its derivative, nucleoside diphosphate immobilized substance which forms a complex with nucleoside diphosphate by substituting phosphate ion of nucleoside triphosphate or its derivative and maintains the binding between nucleoside diphosphate and membrane protein, and a test article; a process of cleaning the membrane fraction expressing the membrane protein with cleaning solvent, and separating at least unreacted labeled nucleoside triphosphate or its derivative by filtration through a filter; and a process of measuring the labeling on the filter and/or the labeling in the filtrate.

2. The screening method for a substance that interacts with ABC protein according to Claim 1, wherein the process of separating by filtration through a filter is a separating process of suction filtration and/or centrifugal filtration through a filter.

3. The screening method for a substance that interacts with ABC protein according to Claim 1 or 2, wherein the nucleoside diphosphate immobilized substance is vanadic acid.

4. The screening method for a substance that interacts with ABC protein according to any one of Claims 1 to 3, wherein the nucleoside triphosphate is ATP.

5. The screening method for a substance that interacts with ABC protein according to any one of Claims 1 to 4, wherein the labeling is radioactive labeling, fluorescent labeling or photoaffinity group labeling.

6. The screening method for a substance that interacts with ABC protein according to Claim 5, wherein the radioactive labeling is ³²P, ³³P, ³⁵S, ¹⁴C, or ³H.

7. The screening method for a substance that interacts with ABC protein according to any one of Claims 1 to 6, wherein the membrane fraction expressing ABC protein is amembrane fraction of a mammal or an insect expressing ABC protein.

8. The screening method for a substance that interacts with ABC protein according to anyone of Claims 1 to 7, wherein the substance that interacts with ABC protein is a substrate for ABC protein.

9. The screening method for a substance that interacts with ABC protein according to anyone of Claims 1 to 7, wherein the substance that interacts with ABC protein is an inhibitor of ABC protein.

10. The screening method for a substance that interacts with ABC protein according to any one of Claims 1 to 9, wherein the ABC protein is a human ABC protein which belongs to ABCA subfamily, ABCB subfamily, ABCC subfamily, ABCD subfamily, ABCE subfamily, ABCF subfamily, or ABCG subfamily.

11. A kit for measuring transporter activity of ABC (ATP Binding Cassette) protein, having a filter which can filtrate and separate a membrane fraction expressing ABC protein, labeled nucleoside triphosphate or its derivative, nucleoside diphosphate immobilized substance which forms a complex with nucleoside diphosphate by substituting phosphate ion of nucleoside triphosphate or its derivative and maintains the binding between nucleoside diphosphate and membrane protein, and at least unreacted labeled nucleoside triphosphate or its derivative.

12. The kit for measuring transporter activity of ABC protein according to Claim 11, wherein the filter which can filtrate and separate is equipped with the means of suction filtration and/or centrifugal filtration.

13. The kit for measuring transporter activity of ABC protein according to Claim 11 or 12, wherein the nucleoside diphosphate immobilized substance is vanadic acid.

14. The kit for measuring transporter activity of ABC protein according to any one of Claims 11 to 13, wherein the nucleoside triphosphate is ATP.

15. The kit for measuring transporter activity of ABC protein according to any one of Claims 11 to 14, wherein the labeling is radioactive labeling, fluorescent labeling or photoaffinity group labeling.

16. The kit for measuring transporter activity of ABC protein according to Claim 15, wherein the radioactive labeling is ³²P, ³³P, ³⁵S, ¹⁴C, or ³H.

17. The kit for measuring transporter activity of ABC protein according to any one of Claims 11 to 16, wherein the membrane fraction expressing ABC protein is a membrane fraction of a mammal or an insect expressing ABC protein.

18. The kit for measuring transporter activity of ABC protein according to any one of Claims 11 to 17, wherein the substance that interacts with ABC protein is a substrate for ABC protein.

19. The kit for measuring transporter activity of ABC protein according to any one of Claims 11 to 17, wherein the substance that interacts with ABC protein is an inhibitor of ABC protein.

20. The kit for measuring transporter activity of ABC protein according to any one of Claims 11 to 19, wherein the ABC protein is a human ABC protein which belongs to ABCA subfamily, ABCB subfamily, ABCC subfamily, ABCD subfamily, ABCE subfamily, ABCF subfamily, or ABCG subfamily.

21. A measuring method for transporter activity of ABC (ATP Binding Cassette) protein, wherein it has the following processes: a process of contacting a membrane fraction expressing ABC protein, labeled nucleoside triphosphate or its derivative, nucleoside diphosphate immobilized substance which forms a complex with nucleoside diphosphate by substituting phosphate ion of nucleoside triphosphate or its derivative and maintains the binding between nucleoside diphosphate and membrane protein, and a substrate of ABC protein; a process of cleaning the membrane fraction expressing the membrane protein with cleaning solvent, and separating at least unreacted labeled nucleoside triphosphate or its derivative by filtration through a filter; and a process of measuring the labeling on the filter and/or the labeling in the filtrate.


SEQUENCE LISTING

<110>  The Circle for the Promotion of Science and Engineering

<120>  Method for Screening Substances interacting with ABC Proteins

<130>  03-057PCT

<150>  JP2003-83686

<151>  2003-03-25

<160>  3

<170>  PatentIn version 3.1

<210>  1

<211>  11

<212>  PRT

<213>   Organism Not Restricted

<220>

<221>   MISC_FEATURE

<222>   (2)..(2)

<223>   Xaa = any amino acid

<220>

<221>   MISC_FEATURE

<222>   (3)..(3)

<223>   Xaa = any amino acid

<220>

<221>   MISC_FEATURE

<222>   (5)..(5)

<223>   Xaa = any amino acid

<400> 1

Gly Xaa Xaa Gly Xaa Gly Lys Ser Ser Thr Gln
1               5                   10

<210> 2
<211> 11
<212> PRT
<213> Organism Not Restricted

<220>
<221> MISC_FEATURE
<222> (7)..(7)
<223> Xaa = any amino acid

<400> 2

Leu Ile Phe Ile Leu Val Xaa Asp Glu Asp Ser
1               5                   10

<210> 3
<211> 93
<212> PRT
<213> Organism Not Restricted

<400> 3

Leu Ile Val Met Phe Tyr Cys Ser Ala Ser Ala Pro Gly Leu Val Phe
1               5                   10                  15

Tyr Lys Gln His Gly Asp Glu Asn Gln Met Trp Lys Arg Gln Ala Ser
            20                  25                  30
Pro Cys Leu Ile Met Phe Trp Lys Arg Asn Gln Ser Thr Ala Val Met
        35                  40                  45
Leu Ile Val Met Phe Tyr Pro Ala Asn Pro His Tyr Leu Ile Val Met
    50                  55                  60
Phe Trp Ser Ala Gly Cys Leu Ile Val Phe Phe Tyr Trp His Pro Lys
65                  70                  75                  80
Arg His Pro Leu Ile Val Met Phe Tyr Trp Ser Thr Ala
                85                  90